# EUROPÄISCHE PATENTANMELDUNG

(11) **EP 1 969 962 A1**
(43) Veröffentlichungstag der Anmeldung: **17.09.2008**
(21) Anmeldenummer: 07023026.3
(22) Anmeldetag: 28.11.2007
(51) Int. Cl.: A45F 5/00, A44C 15/00, A45C 11/18

(54) **Schmuckgegenstand als mobiler Datenträger**

(30) Priorität: 01.12.2006 DE 202006018611 U
(71) Anmelder: Fleischer, Dietmar, 01705 Freital (DE)
(72) Erfinder: Fleischer, Dietmar, 01705 Freital (DE)
(74) Vertreter: Uhlemann, Henry

(57) **Zusammenfassung**

Die Erfindung betrifft Schmuckgegenstände als mobile Datenträger insbesondere für Gesundheitsdaten. Dazu sind zwei plattenförmige Körper (1) über wenigstens einen Steg (2) beabstandet zueinander angeordnet, wobei sich der Datenträger zwischen den plattenförmigen Körpern so befindet, dass dieser wenigstens teilweise herauszieh- oder herausschiebbar ist, und dass wenigstens einer der plattenförmigen Körper (1) eine Öse (4) oder einen Durchbruch besitzt.

## Beschreibung

Die Erfindung betrifft Schmuckgegenstände als mobile Datenträger.

Durch die DE 196 46 636 A1 (Am Körper zu tragende Kleinst-Dokumenten-Kapsel) ist eine Kapsel bekannt, wobei polizeilich relevante Daten zusammen mit physiologischen (körperlichen Eigenheiten) und pathologischen (krankhaften Eigenheiten) Daten in einem Miniaturdokument zusammengefasst sind, welches wegen seiner äußerst geringen Größe permanent am Körper getragen wird, um im Notfall sofort ohne jedwede Verzögerung über die verunglückte Person kompetente Auskunft zu geben. Diese Kleinst-Dokumenten-Kapsel besteht dazu aus zwei Kapseldeckeln, die zusammengedrückt werden können. Dadurch ist ein Behältnis für ein Miniaturdokument vorhanden. Das Miniaturdokument befindet sich dabei lose in diesem Behältnis. Bei einer auch ungewollten Öffnung kann dieses Miniaturdokument leicht unkontrolliert herausfallen und verloren werden.

Der im Patentanspruch 1 angegebenen Erfindung liegt die Aufgabe zugrunde, einen Datenträger einfach in einem Schmuckgegenstand unterzubringen.

Diese Aufgabe wird mit den im Schutzanspruch 1 aufgeführten Merkmalen gelöst.

Die Schmuckgegenstände als mobile Datenträger zeichnen sich insbesondere durch ihre einfache Realisierung aus.

Dazu sind zwei plattenförmige Körper über wenigstens einen Steg beabstandet zueinander angeordnet. Der Datenträger befindet sich so zwischen den plattenförmigen Körpern, dass dieser wenigstens teilweise herauszieh- oder herausschiebbar. Wenigstens einer der plattenförmigen Körper besitzt darüber hinaus eine Öse oder einen Durchbruch.

Die Öse oder der Durchbruch dienen zur Verbindung des Schmuckgegenstandes mit einer Kette. Dadurch kann der Schmuckgegenstand leicht und formschön am Hals oder am Handgelenk befestigt werden. Ein sicheres Tragen des Schmuckgegenstandes mit dem Datenträger ist damit gegeben.

Ein weiterer Vorteil besteht darin, dass der Schmuckgegenstand bei einer durch einen Unfall, einer krankheits- oder alstersbedingten eingeschränkten oder nicht mehr möglichen Bewegung einer Person durch Dritte leicht zugänglich ist. Damit eignet sich der Schmuckgegenstand insbesondere als mobiler Datenträger mit der Gesundheit betreffenden Daten der den Sclunuckgegenstand tragenden Person. Derartige Daten sind zum Beispiel die Person betreffende Daten, wie zum Beispiel der Name, die Wohnung, der Geburtstag, Identifikationsnummern und Familienangehörige, und medizinische Daten, wie zum Beispiel Angaben zum Blut, Vorerkrankungen, Medikamente und Spendenerklärungen.

Ein weiterer Vorteil besteht darin, dass die Daten auch Zugangsdaten sein können. Damit eignet sich der Schmuckgegenstand auch als Schlüssel zur berechtigten Nutzung der unterschiedlichsten Gegenstände oder zum Zugang der unterschiedlichsten Räumlichkeiten in Gebäuden oder in speziellen Aufbewahrungseinrichtungen wie zum Beispiel Schränken oder Truhen.

Der Datenträger befindet sich zwischen den plattenförmigen Körpern, wodurch der Datenträger insbesondere vor mechanischen Beschädigungen geschützt ist.

Bei einer wasserdichten Ausführung des Datenträgers ergeben sich weitere wesentliche Vorteile. So kann der Schmuckgegenstand auch in Verbindung mit Wasser zum Beispiel beim Baden getragen werden.

Die plattenförmigen Körper bestimmen im Wesentlichen den Schmuckcharakter des Schmuckgegenstandes. Dieser wird zum Beispiel durch die eingesetzten Materialien und/oder auf- und/oder eingebrachten Verzierungen bestimmt. Dabei sind die unterschiedlichsten Kombinationen oder Ausgestaltungen möglich.

Dadurch ist ein in den unterschiedlichsten Formen ausgestaltbarer Schmuckgegenstand vorhanden. Damit eignet sich dieser für Person aller Altersstrukturen. Der Datenträger ist universell ausgestalt- und mit den unterschiedlichsten Daten versehbar, so dass leicht eine Anpassung an die persönlichen Wünsche und Bedürfnisse der Träger des Schmuckgegenstandes möglich ist.

Vorteilhafte Ausgestaltungen der Erfindung sind in den Schutzansprüchen 2 bis 16 angegeben.

Der Datenträger ist nach der Weiterbildung des Schutzanspruchs 2 wenigstens eine Blattform eines flächigen Werkstoffes oder eine Platte, wobei das Blatt oder die Platte mit Zeichen, Schrift, bildlichen Darstellungen oder wenigstens einer Kombination davon versehen ist. Die Daten befinden sich damit in schrift- und/oder bildlicher Form auf dem Blatt oder der Platte, so dass die Daten optisch erfasst werden können.

Dazu ist das Blatt oder die Platte in Fortführung nach der Weiterbildung des Schutzanspruchs 3 ein bedruckter Datenträger.

Der Datenträger ist nach der Weiterbildung des Schutzanspruchs 3 eine Platte mit einem Chip. Der Chip ist ein integrierter Schaltkreis mit wenigstens einem Datenspeicher, der lösbar mit einem Datenlesegerät verbindbar ist. Das kann dabei entweder kontaktbehaftet oder kontaktlos erfolgen.

Über die Verbindung mit einem entsprechend ausgeführten Schreibgerät für die Daten des Datenspeichers erfolgt die Datenübertragung.

Dabei können je nach Verwendung des Schmuckgegenstandes die unterschiedlichsten Daten gespeichert werden.

Der Chip ist nach der Weiterbildung des Schutzanspruchs 5 ein Bestandteil eines Transponders oder ein lösbar kontaktierbares Speicherbauelement. Insbesondere ein Transponder als Datenträger ist durch eine kontaktlose Datenübertragung besonders vorteilhaft. Der Transponder ist weiterhin ein passives Element, so dass eine Datenübertragung ständig gewährleistet werden kam. Ein Transponder ist darüber hinaus einfach hand zu haben. Durch ein einfaches beabstandetes Halten vor das Lesegerät werden die Daten des Transponders zum Lesegerät übertragen. Besondere Aufnahmen, Führungen, Bedienelemente oder Kontakte sind nicht notwendig, so dass insbesondere auch jüngere und ältere Personen den Datenträger leicht benutzen können.

Der Datenträger ist nach der Weiterbildung des Schutzanspruchs 6 wenigstens ein Magnetstreifen auf einer Platte. Ein Magnetstreifen ist ein bekannter einfacher Datenträger.

Die plattenförmigen Körper und der Steg sind nach der Weiterbildung des Schutzanspruchs 7 vorteilhafterweise eine U-Form. Die plattenförmigen Körper sind die Schenkel und der Steg das Mittelteil der U-Form. Dadurch ist eine einfache und ökonomische Herstellung des Schmuckgegenstandes gegeben. Der Schmuckgegenstand wird einfach durch zweimaliges Biegen einer Platte realisiert.

Nach der Weiterbildung des Schutzanspruchs 8 besitzen die plattenförmigen Körper an einer der an den Steg angrenzenden Seite jeweils entweder eine Öse oder einen Durchbruch und der Datenträger entweder eine Öse oder einen Durchbruch. Darüber hinaus sind entweder die Ösen oder die Durchbrüche korrespondierend zueinander angeordnet, so dass eine durchgehende Öffnung der Ösen oder der Durchbrüche vorhanden ist. Das ist eine besonders einfach realisierte Verbindung des Datenträgers mit wenigstens einem der plattenförmigen Körper. Dazu wird einfach die Kette durch die Öffnung gezogen, wobei beide Bestandteile über die Kette miteinander verbunden sind. Vorteilhafterweise besitzen beide plattenförmigen Körper eine derartige Öffnung, so dass der Datenträger nur aus den beabstandet zueinander angeordneten plattenförmigen Körpern herausgeschoben werden kann.

Wenigstens eine Kante der plattenförmigen Körper ist nach der Weiterbildung des Schutzanspruchs 9 abgeschrägt. Dadurch kann bei entsprechender Gestaltung der Form des Datenträgers ein einfaches herausziehen oder herausschieben des Datenträgers gewährleistet werden. Dabei überragt ein Bereich des Datenträgers die Schräge, so dass der Datenträger greifbar oder durch Berühren verschiebbar ist.

Nach der Weiterbildung des Schutzanspruchs 10 weist eine Kante des Datenträgers wenigstens eine Erhöhung auf. Diese Erhöhung überragt wenigstens eine Kante eines plattenförmigen Körpers. Damit ist eine Handhabung des Datenträgers leicht möglich. Der Datenträger ist dabei greifbar oder durch Berühren verschiebbar.

Wenigstens eine der sich gegenüberliegenden Innenflächen der plattenförmigen Körper besitzt nach der Weiterbildung des Schutzanspruchs 11 wenigstens bereichsweise eine Schicht aus einem Kunststoff oder einem Gummi. Der Kunststoff oder der Gummi wirken als Federelemente, so dass ein fester Halt des Datenträgers zwischen den beabstandet zueinander angeordneten plattenförmigen Körpern gewährleistet ist.

Wenigstens eine der Außenflächen der plattenförmigen Körper ist nach der Weiterbildung des Schutzanspruchs 12 mit jeweils mindestens einem Zeichen, einer bildlichen Darstellung, einem Symbol oder wenigstens einer Kombination davon versehen. Dadurch ergeben sich vielfältigsten Ausgestaltungen des Schmuckgegenstandes.

Vorteilhafterweise ist nach der Weiterbildung des Schutzanspruchs 13 dazu das Zeichen, die bildliche Darstellung oder das Symbol in die Außenfläche des plattenförmigen Körpers eingraviert.

Günstigerweise bestehen nach der Weiterbildung des Schutzanspruchs 14 die plattenförmigen Körper und der Steg aus wenigstens einem Metall und/oder einem Edelmetall. Neben einer vorteilhaften ästhetischen Ausgestaltung wird bei Verwendung eines Datenträgers mit einem Transponder das ungewollte Auslesen der Daten vermieden. Die plattenförmigen Körper wirken dabei als Abschirmung.

Der flächige Werkstoff ist vorteilhafterweise nach der Weiterbildung des Schutzanspruchs 15 Papier oder ein Kunststoff. Derartige Materialien lassen sich unter anderem leicht bedrucken.

Die Platte des Datenträgers besteht nach der Weiterbildung des Schutzanspruchs 16 aus einem Kunststoff, so dass die Daten bei Verwendung eines elektrisch auslesbaren Datenspeichers ausgelesen werden können.

Ein Ausführungsbeispiel der Erfindung ist in den Zeichnungen jeweils prinzipiell dargestellt und wird im folgenden näher beschrieben.

Es zeigen:
Fig. 1 ein Schmuckgegenstand als mobiler Datenträger in einer Vorderansicht und
Fig. 2 der Schmuckgegenstand in einer Draufsicht.

Ein Schmuckgegenstand als mobiler Datenträger besteht im Wesentlichen aus zwei plattenförmigen Körpern 1, die über wenigstens einen Steg 2 beabstandet zueinander angeordnet sind, und dem Datenträger.

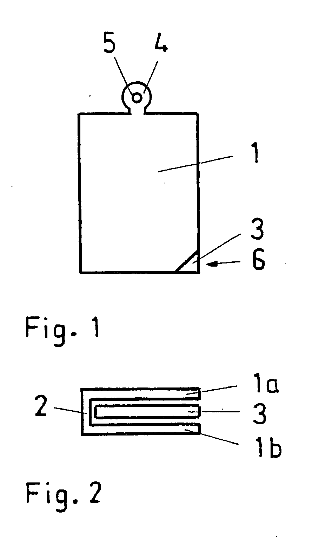

Die Fig. 1 zeigt einen Schmuckgegenstand als mobiler Datenträger in einer Vorderansicht.

Die zwei plattenförmigen Körper 1a, 1b sind über den Steg 2 beabstandet zueinander so angeordnet, dass im Querschnitt eine U-Fonn vorhanden ist. Die Schenkel der U-Fonn werden durch die plattenförmigen Körper 1a, 1b gebildet, während das Mittelteil der Steg 2 ist.

Der Datenträger befindet sich so zwischen den plattenförmigen Körpern 1a, 1b, dass dieser herauszieh- oder herausschiebbar ist. Der Datenträger ist dazu vorteilhafterweise eine Platte 3.
Die Fig. 2 zeigt dazu den Schmuckgegenstand in einer Draufsicht.

Die plattenförmigen Körper 1a, 1b besitzen an einer der an den Steg 2 angrenzenden Seite jeweils eine Öse 4. Auch die Platte 3 als Datenträger weist eine Öse auf. Die Ösen 4 sind korrespondierend zueinander so angeordnet, dass eine durchgehende Öffnung 5 der Ösen 4 vorhanden ist. Mit dem Einbringen einer Kette werden die plattenförmigen Körper 1a, 1b und die Platte 3 als Datenträger vorteilhafterweise miteinander verbunden.

In einer ersten Ausführungsform des Datenträgers ist die Platte 3 mit Zeichen, Schrift, bildlichen Darstellungen oder wenigstens einer Kombination davon versehen. Dazu ist die Platte 3 bedruckt.

In einer zweiten Ausführungsform des Datenträgers ist die Platte 3 mit einem Chip versehen, wobei der Chip als integrierter Schaltkreis wenigstens einen mit einem Datenlesegerät lösbar verbindbaren Datenspeicher besitzt. Der Chip ist Bestandteil eines Transponders oder ein lösbar kontaktierbares Speicherbauelement. Bei dem Transponder ist der Chip mit einer Antenne zusammengeschaltet, die gleichfalls ein Bestandteil der Platte 3 ist.

Zum Herausschieben der Platte 3 kann in einer Ausführungsform wenigstens eine Kante der plattenförmigen Körper 1a, 1b abgeschrägt sein, so dass die Platte 3 als Datenträger die Schrägen 6 überragt und die Platte 3 herausragt (Darstellung in der Fig. 1). In einer weiteren Ausführungsform ist keine Schräge 6 vorhanden, sondern kann die Platte 3 als Datenträger eine Erhöhung aufweisen. Diese Erhöhung überragt die Kanten der plattenförmigen Körper 1a, 1b.

Wenigstens eine der sich gegenüberliegenden Innenflächen der plattenförmigen Körper 1a, 1b kann in einer Ausführungsform wenigstens bereichsweise eine Schicht aus einem Kunststoff oder einem Gummi als Federelement zum Klemmen der zwischen den plattenförmigen Körpern 1a, 1b angeordneten Platte 3 als Datenträger besitzen.

Eine der Außenflächen der plattenförmigen Körper 1a, 1b ist mit jeweils mindestens einem Zeichen, einer bildlichen Darstellung, einem Symbol oder wenigstens einer Kombination davon versehen. Das Zeichen, die bildliche Darstellung oder das Symbol sind dazu in die Außenfläche des plattenförmigen Körpers 1a, 1b eingraviert.

Die plattenförmigen Körper 1a, 1b und der Steg 2 bestehen aus wenigstens einem Metall und/oder einem Edelmetall. Das ist vorteilhafterweise Silber, vergoldetes Silber oder Gold.
Die Platte 3 als Datenträgers besteht vorzugsweise aus einem Kunststoff.

## Patentansprüche

1. Schmuckgegenstand als mobiler Datenträger, **dadurch gekennzeichnet, dass** zwei plattenförmige Körper (1) über wenigstens einen Steg (2) beabstandet zueinander angeordnet sind, dass sich der Datenträger zwischen den plattenförmigen Körpern so befindet, dass dieser wenigstens teilweise herauszieh- oder herausschiebbar ist, und dass wenigstens einer der plattenförmigen Körper (1) eine Öse (4) oder einen Durchbruch besitzt.

2. Schmuckgegenstand nach Schutzanspruch 1, **dadurch gekennzeichnet, dass** der Datenträger wenigstens eine Blattform eines flächigen Werkstoffes ist oder eine Platte (3) und dass das Blatt oder die Platte (3) mit Zeichen, Sclu-ift, bildlichen Darstellungen oder wenigstens einer Kombination davon versehen ist.

3. Schmuckgegenstand nach Schutzanspruch 2, **dadurch gekennzeichnet, dass** das ein bedruckter Datenträger ist.

4. Schmuckgegenstand nach Schutzanspruch 1, **dadurch gekennzeichnet, dass** der Datenträger eine Platte (3) mit einem Chip ist und dass der Chip als integrierter Schaltkreis wenigstens einen mit einem Datenlesegerät lösbar verbindbaren Datenspeicher besitzt.

5. Schmuckgegenstand nach Schutzanspruch 4, **dadurch gekennzeichnet, dass** der Chip ein Bestandteil eines Transponders als Datenträger oder ein lösbar kontaktierbares Speicherbauelement ist.

6. Schmuckgegenstand nach Schutzanspruch 1, **dadurch gekennzeichnet, dass** der Datenträger wenigstens ein Magnetstreifen auf einer Platte (3) ist.

7. Schmuckgegenstand nach Schutzanspruch 1, **dadurch gekennzeichnet, dass** die plattenförmigen Körper (1) und der Steg (2) eine U-Form sind.

8. Schmuckgegenstand nach Schutzanspruch 1 und 7, **dadurch gekennzeichnet, dass** die plattenförmigen Körper (1) an einer der an den Steg (2) angrenzenden Seite jeweils entweder eine Öse (4) oder einen Durchbruch und der Datenträger entweder eine Öse (4) oder einen Durchbruch besitzen, wobei entweder die Ösen (4) oder die Durchbrüche korrespondierend zueinander so angeordnet sind, dass eine durchgehende Öffnung (5) der Ösen (4) oder der Durchbrüche vorhanden ist.

9. Schmuckgegenstand nach Schutzanspruch 1, **dadurch gekennzeichnet, dass** wenigstens eine Kante der plattenförmigen Körper (1) abgeschrägt ist.

10. Schmuckgegenstand nach Schutzanspruch 1, **dadurch gekennzeichnet, dass** eine Kante des Datenträgers wenigstens eine Erhöhung aufweist, wobei die Erhöhung wenigstens eine Kante eines plattenförmigen Körpers (1) überragt.

11. Schmuckgegenstand nach Schutzanspruch 1, **dadurch gekennzeichnet, dass** wenigstens eine der sich gegenüberliegenden Innenflächen der plattenförmigen Körper (1) wenigstens bereichsweise eine Schicht aus einem Kunststoff oder einem Gummi besitzt.

12. Schmuckgegenstand nach Schutzanspruch 1, **dadurch gekennzeichnet, dass** wenigstens eine der Außenflächen der plattenförmigen Körper (1) mit jeweils mindestens einem Zeichen, einer bildlichen Darstellung, einem Symbol oder wenigstens einer Kombination davon versehen ist.

13. Schmuckgegenstand nach Schutzanspruch 12, **dadurch gekennzeichnet, dass** das Zeichen, die bildliche Darstellung oder das Symbol in die Außenfläche des plattenförmigen Körpers (1) eingraviert sind.

14. Schmuckgegenstand nach Schutzanspruch 1, **dadurch gekennzeichnet, dass** die plattenförmigen Körper (1) und der Steg (2) aus wenigstens einem Metall und/oder einem Edelmetall bestehen.

15. Schmuckgegenstand nach Schutzanspruch 2, **dadurch gekennzeichnet, dass** der flächige Werkstoff Papier oder ein Kunststoff ist.

16. Sclunuckgegenstand nach Schutzanspruch 3, **dadurch gekennzeichnet, dass** die Platte (3) als Datenträgers aus einem Kunststoff besteht.
